# EUROPÄISCHE PATENTSCHRIFT

(11) **EP 1 535 055 B1**
(45) Veröffentlichungstag und Bekanntmachung des Hinweises auf die Patenterteilung: **10.12.2008**
(21) Anmeldenummer: 02748532.5
(22) Anmeldetag: 06.08.2002
(51) Int. Cl.: G01N 27/28, G01N 27/38

(54) **VORRICHTUNG UND VERFAHREN ZUM VORBEREITEN EINES ELEKTROCHEMISCHEN SENSORS**
DEVICE AND METHOD FOR PREPARING AN ELECTROCHEMICAL SENSOR
DISPOSITIF ET PROCEDE PERMETTANT DE PREPARER UN DETECTEUR ELECTROCHIMIQUE

(43) Veröffentlichungstag der Anmeldung: 01.06.2005
(73) Patentinhaber: SENTEC AG, 4106 Therwil (CH)
(72) Erfinder: HÄNER, Paul, CH-4144 Arlesheim (CH); LANG, Joseph, F-68220 Ranspach le Haut (FR)
(74) Vertreter: Dr. Graf & Partner
(86) Internationale Anmeldenummer: PCT/CH2002/000431
(87) Internationale Veröffentlichungsnummer: WO 2004/013624

(56) Entgegenhaltungen:
- DE-A- 2 753 698
- DE-A- 4 232 909
- US-A- 4 303 076
- US-A- 4 325 797
- US-A- 4 830 713
- GLASSPOOL W ET AL: "A screen-printed amperometric dissolved oxygen sensor utilising an immobilised electrolyte gel and membrane" SENSORS AND ACTUATORS B, ELSEVIER SEQUOIA S.A., LAUSANNE, CH, Bd. 48, Nr. 1-3, 30. Mai 1998 (1998-05-30), Seiten 308-317, XP004147357 ISSN: 0925-4005

## Beschreibung

Die Erfindung betrifft eine Vorrichtung zum Vorbereiten eines elektrochemischen Sensors gemäss dem Oberbegriff von Anspruch 1 und einen gemeinsemen Träger für die Vorrichtung gemäß Anspruch 14. Die Erfindung betrifft weiter ein Verfahren zum Vorbereiten eines elektrochemischen Sensors gemäss dem Oberbegriff von Anspruch 18.

Es sind elektrochemische Sensoren bekannt, deren Messprinzip auf der lonendiffusion basiert. Diese lonendiffusion erfolgt, unter Verwendung eines geeigneten Elektrolyten, üblicherweise über eine halbdurchlässige Membran, beispielsweise ein Glas für H⁺ und Teflon für CO₂. Derartige elektrochemische Sensoren ermöglichen primär die H⁺-Konzentration zu messen, und indirekt, über die Messung der H⁺-Konzentration, auch die Konzentration von zum Beispiel CO₂ oder O₂ zu bestimmen. Die Messung der H⁺-Konzentration erfolgt mit einer pH-Elektrode, auch als Glaselektrode bezeichnet.

Eine Abwandlung der pH-Elektrode ist die pO₂-Elektrode, auch als Clark-Elektrode bezeichnet, welche zur Messung der O₂-Konzentration dient.

Eine weitere Abwandlung der pH-Elektrode ist die pCO₂-Elektrode, auch als Severinghaus-Elektrode bezeichnet, welche die Messung der CO₂-Konzentration erlaubt. Bei der Severinghaus-Elektrode diffundiert das CO₂ durch eine Membran (Teflon) in einen Elektrolyten mit einer NaHCO₃-Lösung wobei gilt: CO₂ + H₂O <-> H₂CO₃ <-> H⁺ + HCO₃. Die H⁺-Konzentration wird mit der pH-Elektrode gemessen und daraus der CO₂-Wert abgeleitet.

Derartige elektrochemische Sensoren werden unter anderem zur Messung von Blutgaswerten wie der CO₂-Konzentration oder der O₂-Konzentration im Blut verwendet. Der elektrochemische Sensor wird an einer gut durchbluteten Stelle am menschlichen Körper angelegt, um den transkutanen Kohlendioxidpartialdruck (tcpCO₂) beziehungsweise den transkutanen Sauerstoffpatialdruck (tcpO₂) zu messen. Ausführliche Informationen über diese allgemein bekannten Messmethoden sind beispielsweise dem folgenden Übersichtsartikel zu entnehmen: "Noninvasive Assessment of Blood Gases, State of the Art" von J. S. Clark et al., Am. Rev. Resp. Dis., Vol. 145, 1992, pp. 220-232.

Es ist auch bekannt, den elektrochemischen Sensor mit zusätzlichen Sensoren zu versehen, zum Beispiel mit Leuchtdioden und photoelektrischen Sensoren, um zugleich eine pulsoximetrische Messung durchzuführen. Aus der Druckschrift EP 0 267 978 A1 ist ein derartiger Kombinationssensor zur kombinierten Messung der Sauerstoffsättigung des Hämoglobins im arteriellen Blut sowie des arteriellen Kohlendioxidpartialdruckes bekannt. Dieser Kombinationssensor umfasst als elektrochemischer Sensor eine Severinghaus-Elektrode zur Messung des transkutanen CO₂-Partialdrucks, sowie eine Anordnung zur Messung der Sauerstoffsättigung (SpO₂) mittels Pulsoximetrie.

Ein Nachteil elektrochemischer Sensoren ist die Tatsache, dass deren Vorbereitung und Unterhalt sehr anspruchsvoll ist und ausgebildetes Fachpersonal erfordert. Bei der Severinghaus-Elektrode muss beispielsweise die halbdurchlässige Membran und der sich zwischen der Membran und dem Sensorkopf befindliche Elektrolyt regelmässig ausgewechselt werden, um eine einwandfreie Funktion zu gewährleisten. Diese Wartungsarbeit ist sehr anspruchsvoll, da die Messgenauigkeit von der Dicke der Elektrolytschicht sowie von der präzisen Anordnung der Membran abhängig ist. Die Reproduzierbarkeit der Messgenauigkeit ist von zentraler Bedeutung, da mit dem elektrochemischen Sensoren Vitalparameter wie der CO₂-Gehalt im Blut gemessen werden. Eine Fehlmessung dieser Parameter könnte sich für einen Patienten letal auswirken. Der elektrochemische Sensor wird zudem häufig in einem hektischen Umfeld wie einer Intensivstation im Spital verwendet. Der elektrochemische Sensor wird immer öfters jedoch auch im Heimbereich (Home Care) verwendet, um Personen zu Hause zu überwachen.

Die Druckschrift DE 4232909 offenbart ein Verfahren und eine Vorrichtung zur Elektrolytbefüllung bei einem elektrochemischen Sensor. Diese Vorrichtung verwendet eine einstückige Membrankappe mit Membrankammer, wobei die Membran den Boden der Membrankammer bildet, und der Elektrolyt von Hand in die Membrankammer einzufüllen ist.

Ein weiteres Verfahren zum Vorbereiten eines elektrochemischen Sensors ist aus US 4303076 bekannt, wobei der Sensor mit Elektrolyt befüllt und dann mit eines Membrankappe versehen wird.

Es ist daher Aufgabe der vorliegenden Erfindung die Vorbereitung eines elektrochemischen Sensors sicher und einfach zu gestalten.

Diese Aufgabe wird gelöst mit einer Vorrichtung zum Vorbereiten eines elektrochemischen Sensors aufweisend die Merkmale von Anspruch 1. Die Unteransprüche 2 bis 13 betreffen weitere, vorteilhaft ausgestaltete Vorrichtungen. Die Aufgabe wird weiter gelöst mit einem gemeinsamen Träger für die Vorrichtung aufweisend die Merkmale der Ansprüche 14 bis 17. Die Aufgabe wird weiter gelöst mit einem Verfahren zum Vorbereiten eines elektrochemischen Sensors aufweisend die Merkmale von Anspruch 18. Die Unteransprüche 19 bis 21 betreffen weitere, vorteilhafte Verfahrensschritte.

Die Erfindung wird insbesondere gelöst mit einer Vorrichtung zum Vorbereiten eines elektrochemischen Sensors, um dessen Sensorkopf mit einem Elektrolyten und einer Membran zu versehen, umfassend ein Haltemittel für den Sensor, umfassend ein Mittel zur Abgabe des Elektrolyten sowie umfassend ein Mittel zur Abgabe der Membran.

Das Haltemittel, das Mittel zur Abgabe des Elektrolyten sowie das Mittel zur Abgabe der Membran sind gemeinsam in derselben Vorrichtung angeordnet, was den Vorteil aufweist, dass der Sensorkopf reproduzierbar und sehr präzise mit dem Elektrolyt versehen sowie mit der Membran bestückt werden kann. Die Vorrichtung ist derart ausgestaltet, dass der Ablauf des Vorbereitens des Sensors zwangsgeführt ist, indem zuerst der Elektrolyt abzugeben ist, und erst danach der Sensorkopf mit der Membran bestückt werden kann.

Das Haltemittel, das Mittel zur Abgabe des Elektrolyten sowie das Mittel zur Abgabe der Membran sind innerhalb eines gemeinsamen Gehäuses angeordnet, sodass diese während dem Vorbereiten des Sensors nicht von Aussen zugänglich sind.

Das Mittel zur Abgabe der Membran und zum Aufsetzen derselben auf den Sensorkopf weist vorzugsweise eine Feder auf, welche derart angeordnet ist, dass die Membran mit reproduzierbarer Anpresskraft an den Sensorkopf abgegeben und an diesem befestigt wird. Das Mittel zur Abgabe der Membran weist zudem vorteilhafterweise einen Presskörper mit einer Anpressfläche auf, welche während der Abgabe der Membran flächig an der Membran anliegt, um den sich zwischen der Membran und dem Sensorkopf befindlichen Elektrolyten derart reproduzierbar zu verdrängen, dass der mit der Membran verbundene Sensor eine reproduzierbare, insbesondere eine gleichmässige Schichtdicke Elektrolyt zwischen dem Sensorkopf und der Membran aufweist. Diese Vorrichtung weist den entscheidenden Vorteil auf, dass jeder mit der Membran bestückte Sensor somit eine im wesentlichen identische Schichtdicke Elektrolyt aufweist. Dies erhöht die Messsicherheit und reduziert Fehlmessungen. Die erfindungsgemässe Vorrichtung weist den Vorteil auf, dass das Vorbereiten des elektrochemischen Sensors im wesentlichen durch die Eigenschaften der Vorrichtung bestimmt wird, und nicht mehr durch die Erfahrung und das Können einer Fachperson. Somit ist auch in einer Stresssituation, wie sich dies beispielsweise auf einer Intensivstation ergibt, ein sicheres Vorbereiten des elektrochemischen Sensors gewährleistet. Zudem ist es auch für eine unerfahrene Person, beispielsweise im Heimbereich, möglich den Sensor sicher vorzubereiten.

Die erfindungsgemässe Vorrichtung weist in einer bevorzugten Ausgestaltung zudem ein Mittel zum Reinigen des Sensorkopfes sowie ein Mittel zum Entfernen einer alten Membran auf. Ein bereits benutzter Sensor kann somit in die erfindungsgemässe Vorrichtung gesteckt werden, wobei dem Sensor zwangsgeführt vorerst die alte Membran entfernt wird, danach der Sensorkopf gereinigt wird, der Sensorkopf mit Elektrolyt versehen wird ,und abschliessend der Sensor mit einer neuen Membran versehen wird.

Gemäß Anspruch 14 sind alle Verbrauchsteile, welche zum Vorbereiten des elektrochemischen Sensors erforderlich sind, auf einem gemeinsamen Träger angeordnet, wobei dieser Träger als Austauschteil, vorzugsweise als Einweg- oder Wegwerfteil ausgestaltet ist. Dabei ist vor jedem Vorbereiten eines elektrochemischen Sensors vorgängig jeweils ein neuer gemeinsamer Träger in die erfindungsgemässe Vorrichtung einzulegen.

Es ist jedoch auch möglich die gesamte erfindungsgemässe Vorrichtung als Einweg- beziehungsweise Wegwerfteil zu konzipieren, sodass kein gemeinsamer Träger auszutauschen ist. Diese Vorrichtung enthält alle zum Vorbereiten des elektrochemischen Sensors erforderlichen Mittel und muss daher nicht geöffnet werden können. Diese Vorrichtung ist daher besonders einfach zu bedienen.

Die Erfindung wird nachfolgend an Hand von Ausführungsbeispielen beschrieben. Es zeigen:

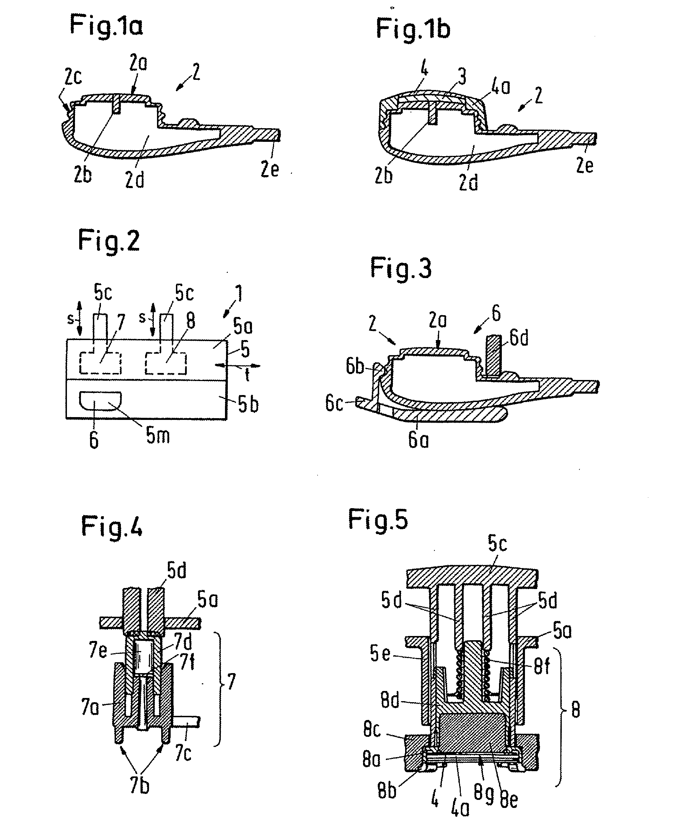

- Fig. 1a: einen Längsschnitt durch einen elektrochemischen Sensor ohne aufgesetzte Membran;
- Fig. 1 b: einen Längsschnitt durch einen elektrochemischen Sensor mit aufgesetzter Membran;
- Fig. 2: eine Seitenansicht einer schematisch dargestellten Vorrichtung zum Vorbereiten eines elektrochemischen Sensors;
- Fig. 3: einen Längsschnitt durch einen in einem Haltemittel gehaltenen Sensor;
- Fig. 4: einen Längsschnitt durch ein Mittel zur Abgabe des Elektrolyten;
- Fig. 5: einen Längsschnitt durch ein Mittel zur Abgabe der Membran;
- Fig. 6: einen Längsschnitt durch ein Mittel zum Reinigen des Sensorkopfes;
- Fig. 7: eine Seitenansicht des Mittels zum Reinigen des Sensorkopfes;
- Fig. 8: einen Längsschnitt durch ein Mittel zum Entfernen der Membran;
- Fig. 9: eine Aufsicht auf einen gemeinsamen Träger;
- Fig. 10: einen Querschnitt durch eine Vorrichtung zum Vorbereiten des elektrochemischen Sensors;
- Fig. 11: eine Seitenansicht des Gehäuses der Vorrichtung zum Vorbereiten des Sensors;
- Fig. 12: eine Aufsicht auf das Gehäuse der Vorrichtung zum Vorbereiten des Sensors;
- Fig. 13: eine perspektivische Ansicht des gemeinsamen Trägers;
- Fig. 14: eine weitere perspektivische Ansicht des gemeinsamen Trägers;
- Fig. 15: eine Teilansicht des Schnittes entlang der Schnittlinie A-A gemäss Fig. 11.

Im folgenden werden für dieselben Gegenstände dieselben Bezugszeichen verwendet.

Figur 1 a zeigt in einem Längsschnitt einen elektrochemischen Sensor 2 mit Sensorkopf 2a, Glaselektrode 2b, Haltenuten 2c, Innenraum 2d und Kabel 2e. Die sich im Innern des Sensors 2 befindlichen Komponenten sind nicht dargestellt. Figur 1b zeigt in einem Längsschnitt den in Figur 1a dargestellten Sensor 2, welcher zur Messung vorbereitet ist, indem dessen Sensorkopf 2a nun mit einem Elektrolyt 3 bedeckt ist, und an dessen Sensorkopf 2a eine Membran 4 mit Haltering 4a befestigt ist. Der in Figur 1b dargestellte elektrochemische Sensor 2 könnte beispielsweise eine Clark-Elektrode oder eine Severinghaus-Elektrode enthalten.

Figur 2 zeigt in einer Seitenansicht eine schematisch dargestellte Vorrichtung 1 zum Vorbereiten des elektrochemischen Sensors 2. Die Vorrichtung umfasst ein Gehäuse 5 bestehend aus einem Gehäuseoberteil 5a sowie einem Gehäuseunterteil 5b, wobei die beiden Gehäuseteile 5a, 5b in Verschiebungsrichtung t gegenseitig verschiebbar gelagert sind. Das Gehäuseunterteil 5b weist eine Öffnung 5m auf, hinter welcher ein Haltemittel 6 für den Sensor 2 angeordnet ist. Im Gehäuseoberteil 5a ist ein Mittel 7 zur Abgabe des Elektrolyten sowie ein Mittel 8 zur Abgabe der Membran angeordnet. Diese beiden Mittel 7,8 sind mit einem Betätigungsmittel 5c gekoppelt, um die Mittel 7,8 in Verschiebungsrichtung s zu bewegen, und dadurch den Elektrolyt 3 oder die Membran 4 dem im Haltemittel 6 fixierten Sensor 2 zuzuführen. Die Mittel 7,8 sind im Innern des Gehäuses 5 angeordnet.

Figur 3 zeigt in einem Längsschnitt einen Sensor 2, welcher im Haltemittel 6, umfassend ein Anschlagteil 6a, ein erstes Halteteil 6b sowie eine Lasche 6c gehalten ist. Der Sensor 2 kann vom Haltemittel 6 gelöst werden, indem die Lasche 6c niedergedrückt wird, und dadurch das erste Halteteil 6b vom Eingriff in den Sensor 2 gelöst wird. Es kann sich als vorteilhaft erweisen, die in Figur 2 dargestellte Öffnung 5m mit einem zweiten Halteteil 6d zu verschliessen, wobei dieses Halteteil 6d derart ausgestaltet ist, dass es auf dem Sensor 2 aufliegt und diesen zusätzlich fixiert.

Figur 4 zeigt einen Längsschnitt durch ein Mittel 7 zur Abgabe des Elektrolyten 3. Das Mittel 7 umfasst einen mit Elektrolyt 7e gefüllten Behälter 7d, welcher einen Verschluss 7f, zum Beispiel mit einer Kugel, aufweist. Der Behälter 7d ist in einem Auslass- und Halteteil 7a gelagert. Das Auslass- und Halteteil 7a kann über einen Steg 7c mit einem gemeinsamen Träger 11 verbunden sein. Ein nach unten auf das Betätigungsmittel 5c ausgeübter Druck wird über den zylinderförmigen Fortsatz 5d auf das Mittel 7 übertragen, sodass dieses vorerst nach unten bewegt wird, bis die Abstandhalter 7b auf dem Sensorkopf 2a aufliegen. Danach wird der Behälter 7d in das Auslass- und Halteteil 7a gepresst, wobei sich der Verschluss 7f bei entsprechend hohem Innendruck öffnet, und der Elektrolyt 7e über den Kanal des Auslass - und Halteteils 7a auf die Oberfläche des Sensorkopfs 2a strömt. Sobald der Druck vom Betätigungsmittel 5c genommen wird, bewegt sich das Mittel 7 wieder nach oben, und die Abstandhalter 7b liegen nicht mehr auf dem Sensorkopf 2a auf.

Figur 5 zeigt einen Längsschnitt durch ein Mittel 8 zur Abgabe der Membran 4. Das Mittel 8 umfasst eine Halterung 8a mit Laschen 8b für den Haltering 4a der Membran 4. Das Mittel 8 umfasst weiter einen Kolben 8d, welcher über das Führungsteil 5e verschiebbar gelagert ist, welcher über eine Feder 8f an das bewegliche Gehäuseoberteil 5c gekoppelt ist, und welcher unten einen Presskörper 8e mit einer Anpressfläche 8g aufweist, welche gleichmässig auf der Membran 4 aufliegt. Das Mittel 8 kann über das Halteteil 8c mit einem gemeinsamen Träger 11 verbunden sein. Der nach unten auf das Betätigungsmittel 5c ausgeübte Druck wirkt über die zylindrischen Fortsätze 5d auf die Feder 8f, sodass der Kolben 8d nach unten bewegt wird. Die Laschen 8b gelangen bei dieser Bewegung in Berührung mit dem Sensor 2 und werden dabei aufgespreizt, sodass die Membran 4 freigegeben ist und daraufhin am Sensor 2 befestigt wird. Während dem Aufsetzen der Membran 4 auf den Sensor 2 liegt, bedingt durch den Presskörper 8e, auf der gesamten Membran 4 eine vorzugsweise gleichmässige Flächenkraft an, sodass der sich zwischen dem Sensorkopf 2a und der Membran 4 befindliche Elektrolyt 3 gleichmässig nach aussen verdrängt wird, bevor die Membran 4 über den Haltering 4a fest mit dem Sensor 2 verbunden wird. Das Mittel 8 weist den Vorteil auf, dass die maximale Anpresskraft des Presskörpers 8e im wesentlichen durch die Feder 8f bestimmt wird, was zur Folge hat, dass die Membran 4 vorzugsweise unabhängig oder kaum abhängig von der auf das bewegliche Gehäuseoberteil 5c bewirkten Kraft mit dem Sensor 2 verbunden wird. Die durch die Feder 8f bewirkte Kraft ist reproduzierbar, was ein gleichmässiges Bespannen des Sensors 2 mit der Membran 4 erlaubt. Der Sensor 2 weist insbesondere eine gleichmässige, reproduzierbare Schichtdicke Elektrolyt 3 auf.

Die in Figur 2 dargestellte Vorrichtung könnte die in den Figuren 3, 4 und 5 dargestellten Mittel 6, 7 und 8 aufweisen, indem das Mittel 6 im Gehäuseunterteil 5b und die Mittel 7 und 8 im Gehäuseoberteil 5a angeordnet sind.

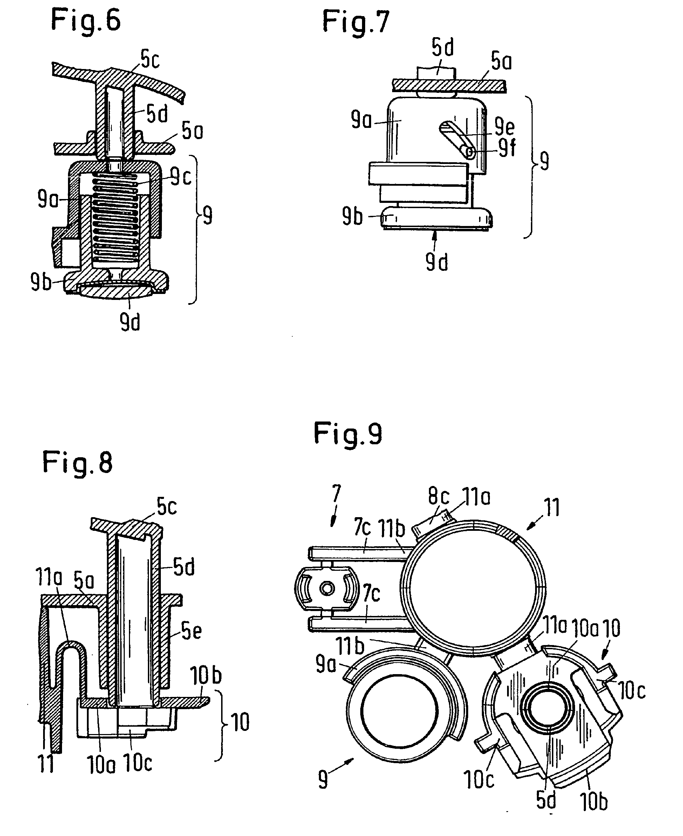

Figur 6 zeigt einen Längsschnitt durch ein Mittel 9 zum Reinigen des Sensorkopfes 2a. Das Mittel 9 umfasst ein Halteteil 9a, in welchem ein Drehkolben 9b mit Reinigungstuch 9d verschiebbar gelagert ist. Der Drehkolben 9b ist zudem über eine Feder 9c an das Halteteil 9a gekoppelt.

Ein nach untern auf das Betätigungsmittel 5c ausgeübter Druck wird über den zylinderförmigen Fortsatz 5d, welcher im Gehäuseoberteil 5a geführt ist, auf das Mittel 9 übertragen, sodass dieses nach unten bewegt wird. Während dieser Bewegung liegt das Reinigungstuch 9d irgendwann auf dem Sensorkopf 2a auf, sodass, auf Grund der weiteren Bewegung, der Drehkolben 9b in das Halteteil 9a hinein gestossen wird. Diese Relativbewegung des Drehkolben 9b bezüglich dem Halteteil 9a bewirkt, wie aus Figur 7 ersichtlich, eine Drehbewegung des Drehkolbens 9b. Figur 7 zeigt in einer Seitensicht das Mittel 9 zum Reinigen des Sensorkopfes 2a. Das Halteteil 9a weist einen schräg verlaufenden Schlitz 9e auf, in welchem eine Nocke 9f, welche mit dem Drehkolben 9b fest verbunden ist, verschiebbar gelagert ist. Liegt nun das Reinigungstuch 9d auf dem Sensorkopf 2a auf, und wird das Halteteil 9a danach weiter nach unten bewegt, so wird der Drehkolben 9b in das Halteteil 9a hinein gestossen, und der Schlitz 9e und die Nocke 9f bewirken eine Drehbewegung des Drehkolbens 9b und damit insbesondere des Reinigungstuchs 9d, sodass der Sensorkopf 2a besonders intensiv gereinigt wird.

Figur 8 zeigt einen Längsschnitt durch ein Mittel 10 zum Entfernen der an einem Sensor 2 befestigten Membran 4. Das Mittel 10 umfasst ein Halteteil 10a, ein Führungsteil 10b sowie ein seitliches Haltemittel 10c. Das Mittel 10 kann über ein federndes Verbindungsmittel 11a mit einem gemeinsamen Träger 11 verbunden sein. Der Sensor 2 mit Membran 4 wird mit nach oben ausgerichteter Membran 4 in das Mittel 10 eingeführt, sodass der Haltering 4a der Membran 4 in den seitlichen Haltemitteln 10c gehalten ist. Daraufhin wird über das Betätigungsmittel 5c, welches über den zylinderförmigen Fortsatz 5d im Gehäuseoberteil 5a sowie dem Führungsteil 5e geführt ist, eine Kraft auf die Membran 4 ausgeübt. Durch diese vom Fortsatz 5d bewirkte Kraft wird der Haltering 4a vom Sensor 2 gelöst. Vorzugsweise ist während diesem Trennen unter dem Mittel 10 das in Figur 3 dargestellte Haltemittel 6 angeordnet, sodass der Sensor 2 unmittelbar nach dem Lösen des Halterings 4a im Haltemittel 6 gehalten wird.

Die in den Figuren 3 bis 8 dargestellten Mittel 6,7,8,9,10 dienen alle zum Vorbereiten eines elektrochemischen Sensors 2. Vorzugsweise sind in einer Vorrichtung 1 zum Vorbereiten des elektrochemischen Sensors 2, wie in Figur 2 dargestellt, zumindest die Mittel 6, 7 und 8 angeordnet. Es kann sich als vorteilhaft erweisen zudem weitere Mittel, wie die Mittel 9 und 10 anzuordnen. All diese Mittel 6,7,8,9,10 können auf unterschiedlichste Weise in einer Vorrichtung 1 angeordnet sein, um ein Vorbereiten des Sensors 2 zu ermöglichen.

Figur 9 zeigt eine Aufsicht eines gemeinsamen Trägers 11, an welchem im Umfangsrichtung verteilt das Mittel 10 zum Entfernen der Membran 4, das Mittel 9 zum Reinigen des Sensorkopfs 2a, das Mittel 7 zur Abgabe des Elektrolyten 3 sowie das Mittel 8 zur Abgabe der Membran 4 angeordnet sind. Vom Mittel 8 ist nur das Halteteil 8c dargestellt.

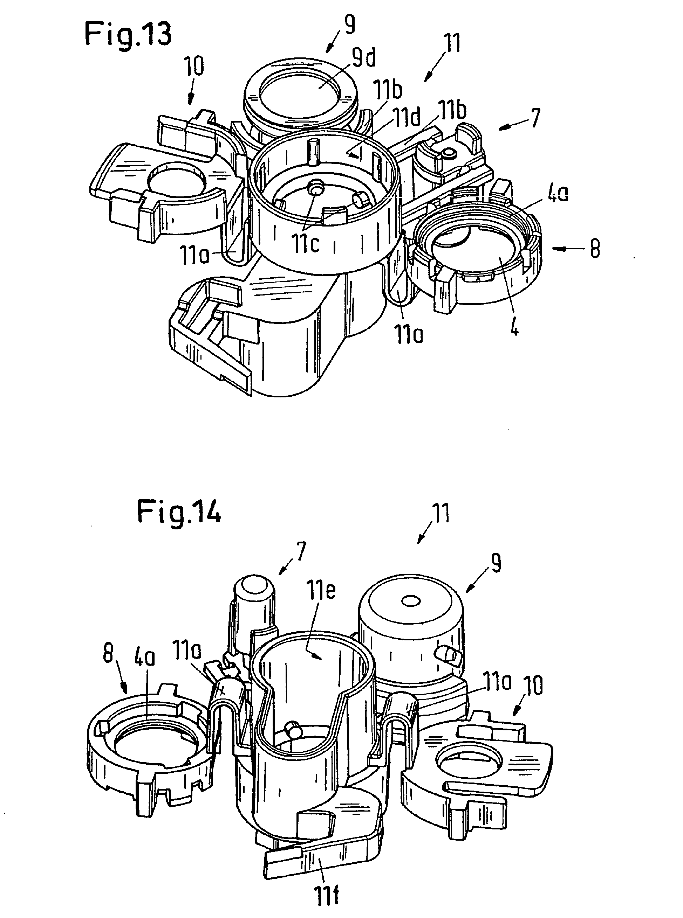

Figur 13 zeigt in einer dreidimensionalen Aufsicht ein weiteres Ausführungsbeispiel eines gemeinsamen Trägers 11 mit in Umfangsrichtung verteilt angeordneten Mitteln 10, 9, 7 und 8. Die transparente Membran 4 mit Haltering 4a ist im Mittel 8 zur Abgabe der Membran gelagert. Der in Figur 13 dargestellte Träger 11 umfasst alle Verbrauchsteile, welche zum Vorbereiten des elektrochemischen Sensors 2 erforderlich sind. Der Träger 11 ist vorzugsweise als Einwegteil beziehungsweise als Wegwerfteil ausgestaltet. Figur 14 zeigt den in Figur 13 dargestellten Träger 11 in einer Untenansicht. Der Träger 11 umfasst federnde Verbindungsmittel 11 a, mit welchen die Mittel 8 und Mittel 10 verbunden sind, sowie starre Verbindungsmittel 11 b, mit welchen die Mittel 7 und 9 verbunden sind. Der Träger 11 umfasst weiter ein erstes Verbindungsteil 11d, Nocken 11c, ein zweites Verbindungsteil 11e sowie ein Arretierteil 11f.

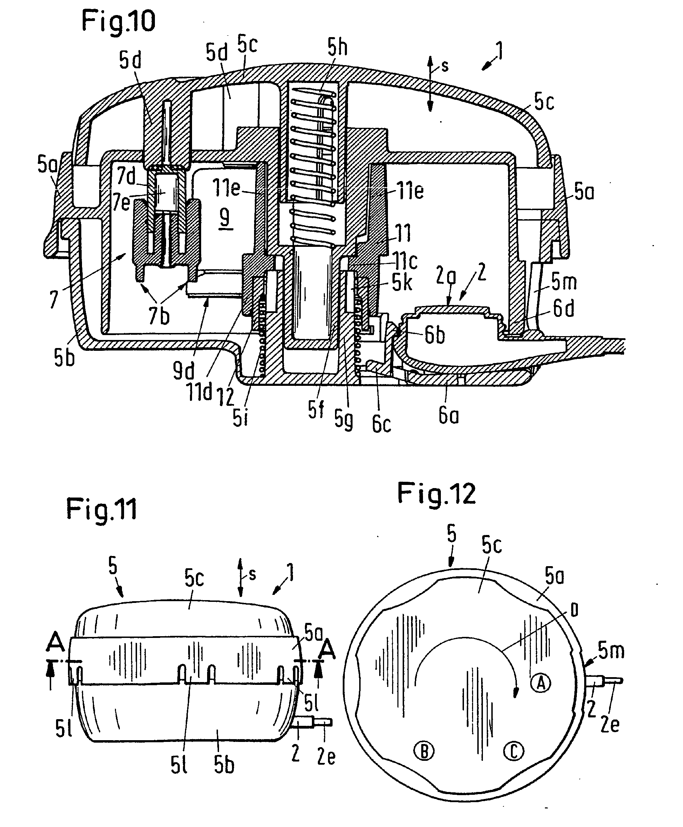

Figur 10 zeigt in einem Querschnitt eine besonders vorteilhaft ausgestaltete Vorrichtung 1 zum Vorbereiten des elektrochemischen Sensors 2. Dieselbe Vorrichtung 1 ist in Figur 11 in einer Seitenansicht und in Figur 12 in der Draufsicht dargestellt. Wie in Figur 11 dargestellt umfasst das Gehäuse 5 ein Gehäuseoberteil 5a sowie ein Gehäuseunterteil 5b, welche zwei Halbschalten bilden, innerhalb welchen der gemeinsame Träger 11 und weitere Komponenten angeordnet sind. Das Gehäuseoberteil 5a weist mehrere, in Umfangsrichtung verteilt angeordnete Haltemittel 51 auf, welche derart in das Gehäuseunterteil 5b eingreifen, dass sich ein Bajonettverschluss ausbildet, dank welchem die beiden Gehäuseteile 5a, 5b gegenseitig verbunden und auch wieder gelöst werden können. Der Bajonettverschluss ist zudem derart ausgestaltet und die Haltemittel 51 sind derart in Umfangsrichtung verteilt angeordnet, dass die beiden Gehäuseteile 5a, 5b gegenseitig drehbar gelagert sind. Das Gehäuse 5 umfasst zudem ein bewegliches Gehäuseoberteil 5c, auch als Betätigungsmittel bezeichnet, welches bezüglich dem Gehäuseoberteil 5a sowie dem Gehäuseunterteil 5b in Bewegungsrichtung s verschiebbar gelagert ist. Ein Teil des Sensors 2 ragt in die Öffnung 5m hinein und befindet sich im Innern des Gehäuses 5. Die Draufsicht gemäss Figur 12 zeigt ebenfalls das Gehäuse 5 mit dem Gehäuseoberteil 5a sowie dem bezüglich diesem verschiebbar gelagerten Betätigungsmittel 5c. Das Gehäuseoberteil 5a ist zudem, zusammen mit dem Betätigungsmittel 5c, bezüglich dem Gehäuseunterteil 5b in Drehrichtung D drehbar gelagert. Das Betätigungsmittel 5c weist an der Oberfläche Markierungen A, B und C auf. Befindet sich die Markierung A bei der Öffnung 5m, so kann der Sensor 2, wie dargestellt, in die Vorrichtung 1 eingeführt werden. Danach wird das Betätigungsmittel 5c in Bewegungsrichtung s nach unten gedrückt und danach nach oben wieder entspannt. Daraufhin wird der Gehäuseoberteil 5a mit dem Betätigungsmittel 5c in Drehrichtung D gedreht, und das Betätigungsmittel 5c wieder in Bewegungsrichtung s nach untern gedrückt und wieder nach oben entspannt. Dieser Vorgang wird wiederholt, bis sich die Markierung B bei der Öffnung 5m befindet. In dieser Stellung des Betätigungsmittels 5c ist die Vorbereitung des Sensors 2 abgeschlossen, und dieser kann aus der Vorrichtung 1 entfernt werden. Durch ein nochmaliges Drehen des Betätigungsmittels 5c in Richtung D gelangt die Markierung C zur Öffnung 5m. In dieser Stellung ist der Bajonettverschluss entriegelt und das Gehäuse 5 kann geöffnet werden. Danach kann der gemeinsame Träger 11 dem Gehäuse 5 entnommen und durch einen neuen gemeinsamen Träger 11 ersetzt werden. Das Gehäuse 5 wird wieder zusammengesetzt und der Bajonettverschluss verriegelt, sodass die Vorrichtung 1 zum Vorbereiten eines weiteren Sensors 2 zur Verfügung steht.

Figur 10 zeigt das Betätigungsmittel 5c, welches bezüglich dem Gehäuseoberteil 5a in Bewegungsrichtung S verschiebbar gelagert ist, wobei sich im Zentrum eine Rückstellfeder 5h befindet, um das Betätigungsmittel 5c nach einer Bewegung nach unten immer wieder in die dargestellte Grundstellung zu bringen. Das Betätigungsmittel 5c umfasst eine Mehrzahl von Stempeln 5d, welche als zylinderförmige Fortsätze ausgebildet sind und welche auf die Mittel 7, 8, 9 oder 10 einwirken. Im Gehäuseunterteil 5b ist das in Figur 3 dargestellte Haltemittel 6 angeordnet, in welchem der Sensor 2 fest gehalten werden kann. Das Gehäuseunterteil 5b umfasst zudem ein Drehlager 5g, in welchem ein Drehzapfen 5f des Gehäuseoberteils 5a drehbar gelagert ist, sodass das Gehäuseoberteil 5a bezüglich dem Gehäuseunterteil 5b in Drehrichtung D drehbar gelagert ist. Im Innenraum des Gehäuses 5 ist der gemeinsame Träger 11 angeordnet. Der gemeinsame Träger 11 ist über ein erstes Verbindungsteil 11d mit dem drehbaren Lager 12 verbunden. Das drehbare Lager 12 wird durch eine Feder 5i nach oben gedrückt. Der Träger 11 ist bezüglich dem Gehäuseunterteil 5b drehbar gelagert. Der Träger 11 ist zudem über ein zweites, exzentrisches Verbindungsteil 11e mit dem Gehäuseoberteil 5a verbunden, und bezüglich einer Drehung in Richtung D an das Gehäuseoberteil 5a gekoppelt. Das Drehlager 5g weist an der Aussenseite in Richtung s verlaufende Nuten 5k auf, in welchen die Nocken 11c während einer Bewegung in Richtung s eingreifen, sodass der gemeinsame Träger 11 sowie das Gehäuseoberteil 5a während dieser Bewegung bezüglich einer Drehung in Richtung D blockiert ist.

In der Vorrichtung 1 gemäss Figur 10 sind alle in den Figuren 3 bis 8 dargestellten Komponenten angeordnet, wobei zur übersichtlicheren Darstellung in Figur 10 nur die Vorrichtung 7 und 9 explizit dargestellt, wogegen die Vorrichtungen 8 und 10 nicht dargestellt sind. Der in Figur 10 angeordnete Träger 11 ist an sich jedoch identisch zu dem in den Figuren 13 und 14 dargestellten Träger 11 und umfasst somit die Vorrichtungen 7, 8, 9 und 10.

Das Vorbereiten eines elektrochemischen Sensors 2 mit der in Figur 10 teilweise dargestellten Vorrichtung 1 umfasst die folgenden Schritte:
- Der Bajonettverschluss der beiden Gehäusehälften 5a, 5b wird geöffnet, der gemeinsame Träger 11 entfernt, ein neuer gemeinsamer Träger 11, wie dieser in den Figuren 13 und 14 dargestellt ist, eingesetzt, der Bajonettverschluss der beiden Gehäusehälften 5a, 5b wieder verschlossen, und die Markierung A des Betätigungsmittels 5c zur Öffnung 5m gedreht.
- Der in Figur 1b dargestellte Sensor 2, umfassend eine Membran 4, wird in die Eintrittsöffnung 5m des Gehäuses 5 eingeführt, wobei sich hinter der Eintrittsöffnung 5m das in Figur 8 dargestellte Mittel 10 zum Entfernen der Membran 4 befindet. Sobald der Sensor 2 in das Mittel 10 eingeführt ist, wird das Betätigungsmittel 5c nach unten gedrückt, sodass der zylinderförmige Fortsatz 5d den Sensor 2 vom Haltering 4a löst, und der Sensor 2, wie in Figur 10 dargestellt, dem Haltemittel 6 zugeführt wird. Der Haltering 4a mit Membran 4 verbleibt im Mittel 10. Daraufhin wird der Druck auf das Betätigungsmittel 5c reduziert oder das Betätigungsmittel 5c vollständig losgelassen, sodass sich das Betätigungsmittel 5c wieder nach oben, in die in Figur 10 dargestellte Grundposition bewegt.
- Daraufhin wird der Gehäusedeckel 5a in Richtung D gedreht, bis sich das Mittel 9 zum Reinigen des Sensorkopfes 2a über dem Sensor 2 befindet. Das Betätigungsmittel 5c wird danach nach unten gedrückt, und der Sensorkopf 2a, wie bereits mit den Figuren 6 und 7 beschrieben, gereinigt, sodass ev. noch auf dem Sensorkopf 2a vorhandener Elektrolyt entfernt wird. Daraufhin wird das Betätigungsmittel 5c wieder losgelassen, sodass sich dieses nach oben bewegt.
- Daraufhin wird der Gehäusedeckel 5a in Richtung D gedreht, bis sich das Mittel 7 zur Abgabe des Elektrolyten über dem Sensor 2 befindet. Das Betätigungsmittel 5c wird danach nach unten gedrückt, und der Sensorkopf 2a, wie bereits mit der Figur 4 beschrieben, mit Elektrolyt 3 versehen. Daraufhin wird das Betätigungsmittel 5c wieder losgelassen, sodass sich dieses nach oben bewegt.
- Daraufhin wird der Gehäusedeckel 5a in Richtung D gedreht, bis sich das Mittel 8 zur Abgabe der Membran 4 über dem Sensor 2 befindet. Das Betätigungsmittel 5c wird danach nach unten gedrückt, und die Membran 4, wie bereits mit der Figur 5 beschrieben, mit dem Sensor 2 verbunden. Daraufhin wird das Betätigungsmittel 5c wieder losgelassen, sodass sich dieses nach oben bewegt.
- Daraufhin wird der Gehäusedeckel 5a in Richtung D gedreht, bis sich die Markierung B vor der Öffnung 5m befindet, sodass der nun vollständig vorbereitete Sensor 2 durch erneutes Drücken und Loslassen des Gehäusedeckels 5a aus der Vorrichtung 1 entfernt werden kann.
- Der Gehäusedeckel 5a wird weiter gedreht, bis sich die Markierung C vor der Öffnung 5m befindet. Der Bajonettverschluss der beiden Gehäusehälften 5a, 5b ist damit entriegelt, der gemeinsame Träger 11 kann entfernt, ein neuer gemeinsamer Träger 11, wie dieser in den Figuren 13 und 14 dargestellt ist, eingesetzt, und der Bajonettverschluss der beiden Gehäusehälften 5a, 5b wieder verschlossen werden.
- Die Vorrichtung 1 ist nun bereit, einen weiteren elektrochemischen Sensor 2 vorzubereiten.

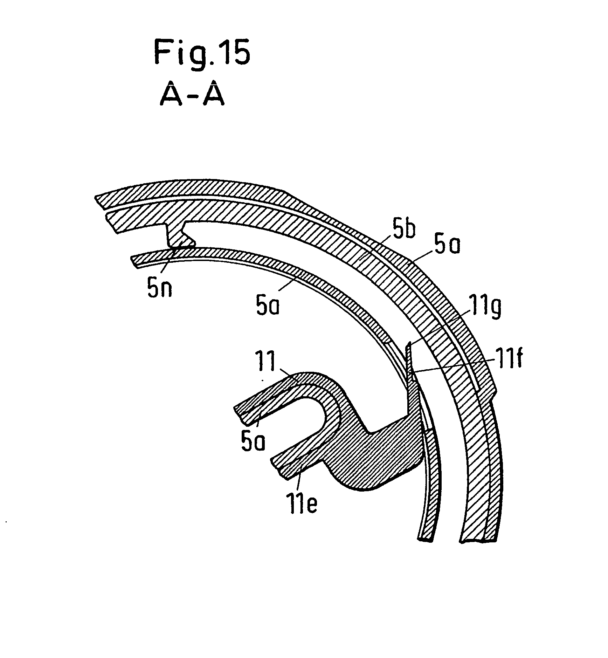

Um beim Drehen des Gehäusedeckels 5a das jeweilige Mittel 7,8,9,10 genau bezüglich dem im Haltemittel 6 gehaltenen Sensor 2 zu positionieren, weist das Gehäuseunterteil 5b, wie in Figur 15 in einem Schnitt A-A entlang der Figur 11 dargestellt, gegen Innen vorstehende Nocken 5n auf, an welchen das Arretierteil 11f des gemeinsamen Trägers 11 mit dessen Zunge 11g jeweils anschlägt. Die Nocken 5n sind derart in Umfangsrichtung verteilt angeordnet, dass beim Anschlagen des Arretierteils 11f das jeweilige Mittel 7,8,9,10 genau derart bezüglich dem Sensor 2 angeordnet ist, sodass durch ein nach unten drücken des Betätigungsmittels 5c die dem Mittel 7,8,9,10 entsprechende Funktion am Sensor 2 ausgeführt werden kann. Die Zunge 11g des Arretierteils 11f ist derart ausgestaltet, dass diese, während dem sich der gemeinsame Träger 11 nach erfolgter Bewegung nach unten wieder nach oben bewegt, unmittelbar nach der Nocke 5n zu liegen kommt, und somit nicht mehr im Eingriff mit der Nocke 5n ist. Damit ist der Gehäusedeckel 5a in Drehrichtung D wieder frei drehbar, bis das Arretierteil 11f in die nächste, vorstehende Nocke 5n eingreift. Diese Ausgestaltung bewirkt, dass die Betätigung der Vorrichtung 1 zwangsgeführt ist, in dem die zu erfolgende Handlung für jeden Schritt vorgegeben ist. Der Gehäusedeckel 5a wird in Drehrichtung D bis zu einem Anschlag gedreht. Danach muss zwangsläufig eine Bewegung des Betätigungsmittels 5c, und damit verbunden eine Bewegung des gemeinsamen Trägers 11 in Bewegungsrichtung s erfolgen. Nachdem sich das Gehäuse 5 wieder in der in Figur 10 dargestellten Grundstellung befindet kann der Gehäusedeckel 5a wiederum in Drehrichtung D bis zum nächstfolgenden Anschlag gedreht werden. Diese Zwangsführung gewährleistet, dass während dem Vorbereiten des Sensors 2 keine Handlung vergessen wird. Die Vorrichtung 1 ist somit besonders geeignet für den Einsatz in einem Umfeld mit hohem Stress, zum Beispiel auf einer Intensivstation, oder für einen Patienten zu Hause, welcher kaum Erfahrung mit der Vorbereitung eines elektrochemischen Sensors 2 aufweist.

Die in den Figuren dargestellten Ausführungsformen von Vorrichtungen zum Vorbereiten eines elektrochemischen Sensors 2 stellen Ausführungsbeispiele dar aus einer Vielzahl von Gestaltungsmöglichkeiten, um die geforderte Funktion des Vorbereitens des Sensors 2 zu erfüllen.

Die in Figur 10 dargestellte Vorrichtung 1 ist auch geeignet, um einen Sensor 2 zum ersten Mal mit einer Membran 4 zu bespannen. Der Sensor 2 ohne Membran 4 und Haltering 4a wird dazu in der in Figur 12 dargestellten Gehäusestellung in die Eintrittöffnung 5m des Gehäuses 5 eingeführt und, durch Drücken des Betätigungsmittels 5c nach unten, mit Hilfe des Mittels 10 im Haltemittel 6 befestigt. Danach wird die Vorrichtung, wie in Figur 10 beschrieben, weiter betätigt, sodass der Sensor 2 mit Elektrolyt und Membran versehen wird. Sobald das Gehäuseoberteil 5a sowie das Betätigungsmittel 5c derart weit gedreht sind, dass sich die Markierung B bei der Eintrittsöffnung 5m befindet, ist der Sensor 2 vollständig vorbereitet.

Die Mittel 7,8,9,10 müssen nicht alle am gemeinsamen Träger 11 angeordnet sein, sondern können beispielsweise auch einzeln oder in Gruppen direkt mit dem Gehäuse 5 verbunden sein.

Vorgängig wurde mehrmals beschrieben, dass das Betätigungsmittel 5c bezüglich der dargestellten Anordnung der Vorrichtung 1 in Richtung nach unten gedrückt wird. Die Vorrichtung 1 kann auch frei in der Hand gehalten werden, sodass das Betätigungsmittel 5c bezüglich dem Gehäuseunterteil 5b nach unten gedrückt wird. Mit unten ist somit nicht die vertikale Richtung zur Erdoberfläche gemeint, sondern eine Bewegung hin zum Gehäuseunterteil 5b.

## Patentansprüche

1. Vorrichtung (1) zum Vorbereiten eines elektrochemischen Sensors (2) um dessen Sensorkopf (2a) mit einem Elektrolyten (3) und einer Membran (4) zu versehen, umfassend ein Mittel zur Abgabe der Membran (8), **dadurch gekennzeichnet, dass** die Vorrichtung ein Haltemittel (6) sowie ein Mittel zur Abgabe des Elektrolyten (7) umfasst, dass das Haltemittel (6), das Mittel zur Abgabe des Elektrolyten (7) sowie das Mittel zur Abgabe der Membran (8) innerhalb eines gemeinsamen Gehäuses (5) angeordnet sind, und dass das Mittel zur Abgabe des Elektrolyten (7) sowie das Mittel zur Abgabe der Membran (8) bezüglich dem Haltemittel (6) verschiebbar gelagert sind.

2. Vorrichtung nach einem der vorhergehenden Ansprüche, **dadurch gekennzeichnet, dass** das Mittel zur Abgabe des Elektrolyten (7) sowie das Mittel zur Abgabe der Membran (8) bezüglich dem Haltemittel (6) verschiebbar gelagert sind, dass das Mittel zur Abgabe des Elektrolyten (7) derart bezüglich dem Haltemittel (6) positionierbar ist, dass der Elektrolyt (3) dem im Haltemittel (6) gehaltenen Sensor (2) zuführbar ist, und dass das Mittel zur Abgabe der Membran (8) derart bezüglich dem Haltemittel (6) positionierbar ist, dass die Membran (4) mit dem im Haltemittel (6) gehaltenen Sensor (2) verbindbar ist.

3. Vorrichtung nach einem der vorhergehenden Ansprüche, **dadurch gekennzeichnet, dass** das Mittel zur Abgabe der Membran (8) derart ausgestaltet ist, dass die Membran (4) mit reproduzierbarer Anpresskraft am Sensorkopf (2a) befestigbar ist.

4. Vorrichtung nach einem der vorhergehenden Ansprüche, **dadurch gekennzeichnet, dass** das Mittel zur Abgabe der Membran (8) einen Presskörper (8e) mit einer Anpressfläche (8g) umfasst, wobei der Presskörper (8e) derart angeordnet ist, dass die Anpressfläche (8g) während der Abgabe der Membran (4) flächig an der Membran (4) anliegt, um den sich zwischen der Membran (4) und dem Sensorkopf (2a) befindlichen Elektrolyten (3) derart zu verdrängen, dass der mit der Membran (4) verbundene Sensor (2) eine reproduzierbare, insbesondere eine gleichmässige Schichtdicke des Elektrolyten (3) zwischen dem Sensorkopf (2a) und der Membran (4) aufweist.

5. Vorrichtung nach einem der vorhergehenden Ansprüche, umfassend zudem ein Mittel zum Reinigen des Sensorkopfes (9), welches bezüglich dem Haltemittel (6) verschiebbar gelagert ist, wobei das Mittel zum Reinigen (9) derart bezüglich dem Haltemittel (6) positionierbar ist, dass dieses den Sensorkopf (2a) des im Haltemittel (6) gehaltenen Sensors (2) mechanisch reinigt.

6. Vorrichtung nach einem der vorhergehenden Ansprüche, umfassend zudem ein Mittel zum Entfernen einer Membran (10), welches bezüglich dem Haltemittel (6) verschiebbar gelagert ist, wobei das Mittel zum Entfernen der Membran (10) derart bezüglich dem Haltemittel (6) positionierbar ist, dass nach dem Entfernen einer gebrauchten Membran (4) der Sensor (2) dem Haltemittel (6) zuführbar ist.

7. Vorrichtung nach einem der vorhergehenden Ansprüche, **dadurch gekennzeichnet, dass** zumindest das Mittel zur Abgabe des Elektrolyten (7) sowie das Mittel zur Abgabe der Membran (8) an einem gemeinsamen Träger (11) befestigt sind, wobei vorzugsweise auch das Mittel zum Reinigen (9) sowie das Mittel zum Entfernen der Membran (10) am gemeinsamen Träger (11) befestigt sind.

8. Vorrichtung nach einem der vorhergehenden Ansprüche, umfassend ein Gehäuse (5) mit einem Gehäuseboden (5b) sowie einem Gehäusedeckel (5a), wobei im Gehäuseboden (5b) das Haltemittel (6) für den Sensor (2) angeordnet ist.

9. Vorrichtung nach Anspruch 8, **dadurch gekennzeichnet, dass** der Gehäuseboden (5b) sowie der Gehäusedeckel (5a) je als Halbschale ausgestaltet sind, welche einen gemeinsamen Innenraum zur Aufnahme von zumindest des Mittels zur Abgabe des Elektrolyten (7) sowie des Mittels zur Abgabe der Membran (8) bilden, und vorzugsweise einen gemeinsamen Innenraum zur Aufnahme aller Mittel (6,7,8,9,10) bildet.

10. Vorrichtung nach Anspruch 9, **dadurch gekennzeichnet, dass** der Gehäuseboden (5b) sowie der Gehäusedeckel (5a) miteinander lösbar verbindbar sind, insbesondere über eine gegenseitige Drehbewegung.

11. Vorrichtung nach einem der Ansprüche 8 bis 10, **dadurch gekennzeichnet, dass** das Gehäuse (5) zudem ein bezüglich dem Gehäusedeckel (5a) beweglich angeordnetes Betätigungsmittel (5c) umfasst, welches eine Wirkverbindung (5d) zu zumindest einem der Mittel (7,8,9,10) aufweist, um über das Betätigungsmittel (5c) eine Kraft und/oder eine Bewegung auf das Mittel (7,8,9,10) zu bewirken.

12. Vorrichtung nach Anspruch 11, **dadurch gekennzeichnet, dass** das Betätigungsmittel (5c) im wesentlichen in bezüglich dem Gehäusedeckel (5a) vertikaler Richtung verschiebbar gelagert ist.

13. Vorrichtung nach einem der Ansprüche 7 bis 12, **dadurch gekennzeichnet, dass** der gemeinsame Träger (11) als Austauschteil, insbesondere als Einwegteil ausgestaltet ist.

14. Gemeinsamer Träger (11) für eine Vorrichtung nach einem der vorhergehenden Ansprüche, umfassend zumindest ein Austauschmittel (7) zur Abgabe eines Elektrolyten aufweisend einen mit Elektrolyt (7e) gefüllten Behälter (7d) sowie umfassend ein separates Austauschmittel (8) zur Abgabe der Membran (4), wobei die Membran (4) im Austauschmittel (8) zur Abgabe den Membran (4) gehalten ist.

15. Gemeinsamer Träger (11) nach Anspruch 14, umfassend zudem ein Mittel zum Reinigen (9) sowie ein Mittel zum Entfernen der Membran (10).

16. Gemeinsamer Träger (11) nach einem der Ansprüche 14 oder 15, **dadurch gekennzeichnet, dass** dieser ein erstes Verbindungsteil (11d) aufweist, welches eine Drehachse definiert, und dass der Behälter (7d) sowie die Membran (4) und insbesondere auch das Mittel zum Reinigen (9) sowie das Mittel zum Entfernen der Membran (10) bezüglich der Drehachse in Umfangsrichtung beabstandet angeordnet sind.

17. Gemeinsamer Träger (11) nach Anspruch 16, **dadurch gekennzeichnet, dass** in Umfangsrichtung nacheinanderfolgend das Mittel zum Entfernen der Membran (10), das Mittel zum Reinigen (9), das Mittel zur Abgabe des Elektrolyten (7) und das Mittel zur Abgabe der Membran (8) angeordnet sind.

18. Verfahren zum Vorbereiten eines elektrochemischen Sensors (2) mit einer Vorrichtung nach einem der Ansprüche 1 bis 13, um dessen Sensorkopf (2a) mit einem Elektrolyten (3) und einer Membran (4) zu versehen, **dadurch gekennzeichnet, dass** der Sensor (2) in einem Haltemittel (6) befestigt wird, und dass daraufhin zwangsgeführt zumindest der Elektrolyt (3) auf den Sensorkopf (2a) aufgebracht wird, und der Sensorkopf (2a) danach mit einer den Elektrolyten (3) bedeckenden Membran (4) versehen wird.

19. Verfahren nach Anspruch 18, **dadurch gekennzeichnet, dass** die Membran (4) mit einer durch eine Feder definierte, reproduzierbare Anpresskraft dem Sensorkopf (2a) zugeführt wird, um den zwischen der Membran (4) und dem Sensorkopf (2a) befindlichen Elektrolyten (3) derart reproduzierbar zu verdrängen, das zwischen der Membran (4) und dem Sensorkopf (2a) jeweils eine reproduzierbare Schichtdicke Elektrolyt (3) entsteht.

20. Verfahren nach Anspruch 18 oder 19, **dadurch gekennzeichnet, dass** zwangsgeführt vorerst die ursprüngliche Membran vom Sensorkopf (2a) entfernt wird, danach der Sensorkopf (2a) gereinigt wird, danach Elektrolyt (3) auf dem Sensorkopf (2a) abgelagert wird, und danach eine Membran (4) mit dem Sensor (2) verbunden wird.

21. Verfahren nach Anspruch 20, **dadurch gekennzeichnet, dass** die Zwangsführung derart erfolgt, dass der Sensor (2) innerhalb eines Gehäuse (5) befestigt wird, und dass durch Drehen eines Teils des Gehäuses (5) die einzelnen Schritte am Sensor (2) zwangsgeführt erfolgen.

## Claims

1. Apparatus (1) for the preparation of an electrochemical sensor (2) in order to provide its sensor head (2a) with an electrolyte (3) and a membrane (4), the apparatus comprising a means for dispensing the membrane (8), **characterized in that** the sensor comprises a holding means (6) as well as a means for dispensing the electrolyte (7), that the holding means (6), the means for dispensing the electrolyte (7) and also the means for dispensing the membrane (8) are arranged within a common housing (5) and **in that** the means for dispensing the electrolyte (7) and also the means for dispensing the membrane (8) are displaceably mounted with respect to the holding means (6).

2. Apparatus in accordance with one of the preceding claims, **characterized in that** the means for dispensing the electrolyte (7) and also the means for dispensing the membrane (8) are displaceably mounted with respect to the holding means (6), **in that** the means for dispensing the electrolyte (7) is positionable with respect to the holding means (6) such that the electrolyte (3) can be supplied to the sensor (2) held in the holding means (6) and **in that** the means for dispensing the membrane (8) can be positioned with respect to the holding means (6) in such a way that the membrane (4) can be connected to the sensor (2) held in the holding means (6).

3. Apparatus in accordance with one of the preceding claims, **characterized in that** the means for dispensing the membrane (8) is designed in such a way that the membrane (4) can be secured to the sensor head (2a) with a reproducible pressing force.

4. Apparatus in accordance with one of the preceding claims, **characterized in that** the means for dispensing the membrane (8) includes a pressing body (8e) with a pressing surface (8g), wherein the pressing body (8e) is arranged such that the pressing surface (8g) contacts the membrane (4) in areal manner during the dispensing of the membrane (4) in order to displace electrolyte located between the membrane (4) and the sensor head (2a) in such a way that the sensor connected to the membrane (4) has a reproducible layer thickness of the electrolyte (3), in particular a uniform a layer thickness of the electrolyte (3), between the sensor head (2a) and the membrane (4).

5. Apparatus in accordance with one of the preceding claims, comprising in addition a means for the cleaning of the sensor head (9) which is displaceably mounted with respect to the holding means (6), wherein the means for cleaning (9) can be so positioned with respect to the holding means (6) that it mechanically cleans the sensor head (2a) of the sensor (2) held in the holding means (6).

6. Apparatus in accordance with one of the preceding claims, comprising in addition a means for the removal of a membrane (10), which is displaceably mounted with respect to the holding means (6), wherein the means for the removal of the membrane (10) can be positioned with respect to the holding means (6) such that after the removal of a used membrane (4) the sensor (2) can be supplied to the holding means (6).

7. Apparatus in accordance with one of the preceding claims, **characterized in that** at least the means for the dispensing of the electrolyte (7) and also the means for the dispensing of the membrane (8) are secured to a common carrier (11), wherein the means for the cleaning (9) and also the means for the removal of the membrane (10) are preferably also secured to the common carrier (11).

8. Apparatus in accordance with one of the preceding claims, comprising a housing (5) with a housing base (5b) and also a housing cover (5a) wherein the holding means (6) for the sensor (2) is arranged in the housing base (5b).

9. Apparatus in accordance with claim 9, **characterized in that** the housing base (5b) and also the housing cover (5a) are each designed as a half shell which form a common inner space for the reception of at least the means for the dispensing of the electrolyte (7) and also of the means for the dispensing of the membrane (8) and preferably form a common inner space for the reception of all means (6, 7, 8, 9, 10).

10. Apparatus in accordance with claim 10, **characterized in that** the housing base (5b) and also the housing cover (5a) are releasably connectable to one another, in particular by a mutual rotary movement.

11. Apparatus in accordance with one of the claims 9 to 11, **characterized in that** the housing (5) also includes an actuating means (5c) movably disposed with respect to the housing cover (5a) which has an operative connection (5d) to at least one of the means (7, 8, 9, 10) in order to bring about a force and/or a movement on the means (7, 8, 9, 10) via the actuating means (5c).

12. Apparatus in accordance with claim 12, **characterized in that** the actuating means (5c) is displaceably mounted essentially in the vertical direction with respect to the housing cover (5a).

13. Apparatus in accordance with one of the claims 8 to 13, **characterized in that** the common carrier (11) is formed as an exchangeable part, and in particular as a disposable part.

14. Common carrier (11) for an apparatus in accordance with one of the preceding claims, comprising at least an exchangeable means (7) for dispensing of an electrolyte comprising a container (7d) filled with electrolyte (7e), and also comprising a separate exchangeable means (8) for dispensing of the membrane (4), wherein the membrane (4) is hold in the exchangeable means (8) for dispensing the membrane (4).

15. Common carrier (11) in accordance with claim 14, comprising in addition, a means for the cleaning (9) and also a means for the removal of the membrane (10).

16. Common carrier (11) in accordance with one of the claims 14 or 15, **characterized in that** it has a first connection part (11d) which defines an axis of rotation and **in that** the container (7d) and also the membrane (4) and in particular also the means for cleaning (9) and also the means for the removal of the membrane (10) are arranged spaced apart in a peripheral direction with respect to the axis of rotation.

17. Common carrier (11) in accordance with 16, **characterized in that** the means for the removal of the membrane (10), the means for cleaning (9), the means for the dispensing of the electrolyte (7) and the means for the dispensing of the membrane (8) are arranged following one another in the peripheral direction.

18. Method for the preparation of an electrochemical sensor (2) with an apparatus according to one of claims 1 to 13, in order to provide a sensor head (2a) with an electrolyte (3) and a membrane (4), **characterized in that** the sensor (2) is secured in a holding means (6) and **in that** thereafter, in compulsory guided manner, at least the electrolyte (3) is applied onto the sensor head (2a) and the sensor head (2a) is then provided with a membrane (4) which covers the electrolyte (3).

19. Method in accordance with claim 18, **characterized in that** the membrane (4) is supplied to the sensor head (2a) with a reproducible pressing force defined by a spring in order to reproducibly displace electrolyte (3) present between the membrane (4) and the sensor head (2a) in such a way that in each case a reproducible layer thickness of electrolyte (3) arises between the membrane (4) and the sensor head (2a).

20. Method in accordance with claim 18 or 19, **characterized in that**, compulsory guided, the original membrane is first removed from the sensor head (2a), the sensor head (2a) is thereafter cleaned, the electrolyte (3) is thereafter deposited on the sensor head (2a) and a membrane (4) is thereafter connected to the sensor (2).

21. Method in accordance with claim 20, **characterized in that** the compulsory guidance takes place in such a way that the sensor (2) is secured within a housing (5) and **in that** the individual steps at the sensor (2) are compulsorily guided by rotation of a part of the housing (5).

## Revendications

1. Dispositif (1) permettant de préparer un capteur électrochimique (2) en vue de munir sa tête de détection (2a) d'un électrolyte (3) et d'une membrane (4), comprenant un moyen de distribution de la membrane (8), **caractérisé en ce que** le dispositif comprend un moyen de maintien (6) ainsi qu'un moyen de distribution de l'électrolyte (7), que le moyen de maintien (6), le moyen de distribution de l'électrolyte (7) ainsi que le moyen de distribution de la membrane (8) sont disposés à l'intérieur d'un boîtier commun (5), et que le moyen de distribution de l'électrolyte (7) ainsi que le moyen de distribution de la membrane (8) sont montés déplaçables par rapport au moyen de maintien (6).

2. Dispositif selon l'une des revendications précédentes, **caractérisé en ce que** le moyen de distribution de l'électrolyte (7) ainsi que le moyen de distribution de la membrane (8) sont montés déplaçables par rapport au moyen de maintien (6), que le moyen de distribution de l'électrolyte (7) est positionnable par rapport au moyen de maintien (6) de façon que l'électrolyte (3) puisse être amené au capteur (2) maintenu dans le moyen de maintien (6), et que le moyen de distribution de la membrane (8) est positionnable par rapport au moyen de maintien (6) de façon que la membrane (4) puisse être reliée au capteur (2) maintenu dans le moyen de maintien (6).

3. Dispositif selon l'une des revendications précédentes, **caractérisé en ce que** le moyen de distribution de la membrane (8) est conçu de façon que la membrane (4) puisse être fixée avec une force de pression reproductible à la tête de détection (2a).

4. Dispositif selon l'une des revendications précédentes, **caractérisé en ce que** le moyen de distribution de la membrane (8) comprend un corps de pression (8e) avec une surface de pression (8g), le corps de pression (8e) étant disposé de façon que la surface de pression (8g) soit appliquée à plat sur la membrane (4) pendant la distribution de la membrane (4) pour refouler l'électrolyte (3) se trouvant entre la membrane (4) et la tête de détection (2a) de façon que le capteur (2) relié à la membrane (4) présente une épaisseur de couche reproductible, en particulier uniforme de l'électrolyte (3) entre la tête de détection (2a) et la membrane (4).

5. Dispositif selon l'une des revendications précédentes, comprenant en outre un moyen de nettoyage de la tête de détection (9), lequel est monté déplaçable par rapport au moyen de maintien (6), le moyen de nettoyage (9) étant positionnable par rapport au moyen de maintien (6) de façon qu'il nettoie mécaniquement la tête de détection (2a) du capteur (2) maintenu dans le moyen de maintien (6).

6. Dispositif selon l'une des revendications précédentes, comprenant en outre un moyen d'enlèvement d'une membrane (10), lequel est monté déplaçable par rapport au moyen de maintien (6), le moyen d'enlèvement de la membrane (10) étant positionnable par rapport au moyen de maintien (6) de façon qu'après l'enlèvement d'une membrane (4) usagée, le capteur (2) puisse être amené au moyen de maintien (6).

7. Dispositif selon l'une des revendications précédentes, **caractérisé en ce qu'**au moins le moyen de distribution de l'électrolyte (7) ainsi que le moyen de distribution de la membrane (8) sont fixés à un support commun (11), le moyen de nettoyage (9) et le moyen d'enlèvement de la membrane (10) étant de préférence aussi fixés au support commun (11).

8. Dispositif selon l'une des revendications précédentes, comprenant un boîtier (5) avec un fond de boîtier (5b) ainsi qu'un couvercle de boîtier (5a), le moyen de maintien (6) pour le capteur (2) étant disposé dans le fond de boîtier (5b).

9. Dispositif selon la revendication 8, **caractérisé en ce que** le fond de boîtier (5b) ainsi que le couvercle de boîtier (5a) sont réalisés sous la forme de demi-coques qui forment un espace intérieur commun pour recevoir au moins le moyen de distribution de l'électrolyte (7) ainsi que le moyen de distribution de la membrane (8), et de préférence un espace intérieur commun pour recevoir tous les moyens (6, 7, 8, 9, 10).

10. Dispositif selon la revendication 9, **caractérisé en ce que** le fond de boîtier (5b) et le couvercle de boîtier (5a) peuvent être reliés l'un à l'autre de manière détachable, en particulier par un mouvement de rotation mutuelle.

11. Dispositif selon l'une des revendications 8 à 10, **caractérisé en ce que** le boîtier (5) comprend en outre un moyen d'actionnement (5c) disposé de manière mobile par rapport au couvercle de boîtier (5a), lequel présente une liaison fonctionnelle (5d) avec au moins un des moyens (7, 8, 9, 10) pour induire par l'intermédiaire du moyen d'actionnement (5c) une force et/ou un mouvement sur le moyen (7, 8, 9, 10).

12. Dispositif selon la revendication 11, **caractérisé en ce que** le moyen d'actionnement (5c) est monté déplaçable essentiellement en direction verticale par rapport au couvercle de boîtier (5a).

13. Dispositif selon l'une des revendications 7 à 12, **caractérisé en ce que** le support commun (11) est réalisé sous forme de pièce échangeable, en particulier sous forme de pièce à usage unique.

14. Support commun (11) pour un dispositif selon l'une des revendications précédentes, comprenant au moins un moyen échangeable (7) de distribution d'un électrolyte présentant un récipient (7d) rempli d'électrolyte (7e) ainsi que comprenant un moyen échangeable séparé (8) de distribution de la membrane (4), la membrane (4) étant maintenue dans le moyen échangeable (8) de distribution de la membrane (4).

15. Support commun (11) selon la revendication 14, comprenant en outre un moyen de nettoyage (9) ainsi qu'un moyen d'enlèvement de la membrane (10).

16. Support commun (11) selon l'une des revendications 14 ou 15, **caractérisé en ce que** celui-ci présente une première pièce de liaison (11d) qui définit un axe de rotation, et que le récipient (7d) ainsi que la membrane (4) et en particulier aussi le moyen de nettoyage (9) et le moyen d'enlèvement de la membrane (10) sont disposés à distance en direction périphérique par rapport à l'axe de rotation.

17. Support commun (11) selon la revendication 16, **caractérisé en ce que** le moyen d'enlèvement de la membrane (10), le moyen de nettoyage (9), le moyen de distribution de l'électrolyte (7) et le moyen de distribution de la membrane (8) sont disposés de manière successive en direction périphérique.

18. Procédé permettant de préparer un capteur électrochimique (2) avec un dispositif selon l'une des revendications 1 à 13 en vue de munir sa tête de détection (2a) d'un électrolyte (3) et d'une membrane (4), **caractérisé en ce que** le capteur (2) est fixé dans un moyen de maintien (6), et qu'après cela au moins l'électrolyte (3) est appliqué par guidage forcé sur la tête de détection (2a), et que la tête de détection (2a) est ensuite munie d'une membrane (4) recouvrant l'électrolyte (3).

19. Procédé selon la revendication 18, **caractérisé en ce que** la membrane (4) est amenée à la tête de détection (2a) avec une force de pression reproductible définie par un ressort, pour refouler de manière reproductible l'électrolyte (3) se trouvant entre la membrane (4) et la tête de détection (2a) de façon que soit formée à chaque fois une épaisseur de couche reproductible d'électrolyte (3) entre la membrane (4) et la tête de détection (2a).

20. Procédé selon la revendication 18 ou 19, **caractérisé en ce que**, par guidage forcé, d'abord la membrane initiale est enlevée de la tête de détection (2a), ensuite la tête de détection (2a) est nettoyée, ensuite l'électrolyte (3) est déposé sur la tête de détection (2a) et ensuite une membrane (4) est reliée au capteur (2).

21. Procédé selon la revendication 20, **caractérisé en ce que** le guidage forcé est réalisé en fixant le capteur (2) à l'intérieur d'un boîtier (5) et en exécutant les différentes étapes sur le capteur (2) par guidage forcé en faisant tourner une partie du boîtier (5).
